# FASCICULE DE BREVET EUROPEEN

(11) **EP 3 981 377 B1**
(45) Date de publication et mention de la délivrance du brevet: **28.06.2023**
(21) Numéro de dépôt: 20306178.3
(22) Date de dépôt: 08.10.2020
(51) Int. Cl.: A61K 8/02, A61K 8/73, A61K 8/9789, A61Q 19/00, A61Q 19/08

(54) **PROCEDE COSMETIQUE**
KOSMETISCHES VERFAHREN
COSMETIC PROCESS

(43) Date de publication de la demande: 13.04.2022
(73) Titulaire: Millier, Claire, 75018 Paris (FR)
(72) Inventeur: Millier, Claire, 75018 Paris (FR)
(74) Mandataire: Novagraaf Technologies

(56) Documents cités:
- CN-A- 111 346 043
- FR-A1- 2 385 160
- DATABASE GNPD [Online] MINTEL; 10 avril 2018 (2018-04-10), anonymous: "Urban Cream SPF 30", XP055590276, Database accession no. 5552271

## Description

### Domaine technique

La présente invention se rapporte à un procédé de traitement cosmétique non thérapeutique comprenant une étape d'application sur la peau du visage d'une composition cosmétique.

La présente invention trouve une application dans le domaine de la cosmétique.

Dans la description ci-dessous, les références entre crochets ([ ]) renvoient à la liste des références présentée à la fin du texte.

### Etat de la technique

Qu'il soit chronique ou épisodique, la peau subit les effets négatifs du stress : perte d'éclat, marques de fatigue, rides, eczéma, boutons, rougeurs, cernes.

Pour comprendre comment cette manifestation de notre tension nerveuse peut agir sur notre peau, il faut remonter au niveau embryonnaire. En effet, la peau et le système nerveux proviennent des mêmes tissus, et continuent à communiquer tout au long de la vie, principalement par le biais des systèmes endocrinien et immunitaire.

Lors d'une situation de stress soudain et intense, le système nerveux envoie une série de signaux relayés par des messagers chimiques ou neuromédiateurs. Cortisol et adrénaline, entre autres, sont alors libérés dans l'organisme. Ces substances vont permettre de mobiliser toute l'énergie disponible pour échapper à un danger par exemple. Dans ces moments-là, priorité absolue est donnée à nos organes vitaux et à nos muscles. Mais lorsque le stress perdure et devient chronique, la peau en fait les frais. Moins bien alimentée que les organes vitaux, elle se déshydrate, se régénère moins bien et se fragilise. Le cortisol quant à lui, affecte son système immunitaire et contribue à la dégradation du collagène, facilitant d'une part l'installation de diverses pathologies cutanées, accélérant d'autre part le vieillissement de notre peau.

Il existe actuellement des soins cosmétiques qui promettent de désensibiliser la peau et de la rendre plus résistante face au stress. Ces soins sont avant tout de bons hydratants qui rétablissent la fonction barrière de la peau.

D'autres soins cosmétiques incorporent des huiles essentielles aux propriétés relaxantes pour aider à évacuer le stress.

Il existe également des produits cosmétiques contenant des actifs qui sont capables de moduler les déséquilibres dus au stress et de calmer l'inflammation. CN 111 346 043 A (JIANG HUIQIN) 30 juin 2020 décrit un procédé de traitement cosmétique comprenant une étape d'application d'une composition cosmétique comprenant de l'acide hyaluronique et un extrait de fleur de Sophora Japonica.

Toutefois, d'autres solutions sont souhaitables, afin de proposer des solutions alternatives et efficaces aux problèmes esthétiques causés par le stress.

### Description de l'invention

La présente invention a précisément pour but de répondre à ces besoins et inconvénients en fournissant un procédé de traitement cosmétique non thérapeutique alliant l'efficacité d'actifs cosmétiques à la stimulation positive du mental, permettant de mettre au service de la peau l'énergie mobilisatrice de la projection mentale induite par le procédé de l'invention.

Avantageusement, la mise en oeuvre des étapes du procédé de l'invention permet de mettre en synergie la peau et le mental, et ainsi d'obtenir une amélioration de l'état de la peau qui est supérieure à celle obtenue par la simple application d'une composition cosmétique, sans stimulation positive du mental. De manière surprenante, les résultats obtenus par la mise en oeuvre du procédé de l'invention sont une diminution des rides du visage, des traits du visage plus détendus, et une peau mieux hydratée.

Ainsi, un premier objet de l'invention se rapporte à un procédé de traitement cosmétique non thérapeutique comprenant une étape d'application sur la peau du visage d'une composition cosmétique et au moins une étape de sophrologie, ladite composition cosmétique comprenant :
- de l'acide hyaluronique,
- un extrait de fleur de Sophora Japonica, et
- optionnellement un hydrolat de rose,
l'étape de sophrologie comprenant les étapes successives suivantes :
(a) adopter une position corporelle allongée ou semi-allongée,
(b) appliquer la composition cosmétique sous forme de masque sur le visage,
(c) décompter en respirant,
(d) réaliser des respirations abdominales, selon un rythme de 5 secondes à l'inspiration et de 5 secondes à l'expiration,
(e) détendre chaque partie du corps de haut en bas pour entrer dans un « état sophroliminal ».

L'acide hyaluronique peut être un produit disponible dans le commerce et adapté à la présente utilisation, comme par exemple Shandong Focus chem Biot ech Co., Ltd sous sa forme pure ou sous forme d'hyaluronate de sodium. Il peut s'agir notamment d'acide hyaluronique de haut poids moléculaire, ou d'acide hyaluronique de poids intermédiaire, ou d'acide hyaluronique de bas poids moléculaire, ou un mélange de deux ou trois types d'acide hyaluronique. Avantageusement, la composition cosmétique peut comprendre, en pourcentage en poids par rapport au poids total de celle-ci, entre 0,01 % et 10,0% d'acide hyaluronique, par exemple entre 0,01 et 9,0%, ou entre 0,01 et 5,0%, ou entre 0,05 et 0,45 %, ou entre 0,10 et 0,40 %, par exemple environ 0,30%. Avantageusement, l'acide hyaluronique permet de retenir l'eau dans la peau et d'améliorer son élasticité.

L'extrait de fleur de Sophora Japonica peut être un produit disponible dans le commerce et adapté à la présente utilisation, comme par exemple Résistress de Solabia, ou être obtenu par toute méthode connue de l'homme du métier, par exemple par extraction aqueuse, alcoolique ou hydroalcoolique. Avantageusement, la composition cosmétique peut comprendre, en pourcentage en poids par rapport au poids total de celle-ci, entre 0,1% et 5,0% de l'extrait de fleur de Sophora Japonica, par exemple entre 0,1 et 4,0%, ou entre 0,1 et 3,0%, ou entre 0,1 et 2,0%, ou entre 0,1 et 0,9%, ou entre 0,3 et 0,7%, par exemple environ 0,5%. Avantageusement, l'extrait de fleur de Sophora Japonica peut stimuler les mécanismes d'auto-défense de la peau.

L'hydrolat de rose peut être un produit disponible dans le commerce et adapté à la présente utilisation, comme par exemple PFVRELROSE14 de Herbarom Laboratoire, ou être obtenu par toute méthode connue de l'homme du métier, par exemple par infusion de boutons et/ou de pétales de roses dans de l'eau distillée. Lorsque l'hydrolat de rose est présent, son pourcentage en poids par rapport au poids total de celle-ci peut être compris entre 0,01% et 50,0%, par exemple entre 0,01% et 10,0%, ou entre 0,01 et 5,0%, ou entre 0,5 et 3,0%, ou entre 0,5 et 2,0%, par exemple environ 1,0%. Avantageusement, l'hydrolat de rose a des effets apaisants pour la peau.

On entend par « composition cosmétique », dans la présente invention, toute composition à visée cosmétique, c'est à dire esthétique, pouvant être mise en contact avec les parties superficielles du corps humain, notamment l'épiderme. Avantageusement, une composition cosmétique permet, exclusivement ou principalement, de les protéger, maintenir en bon état, modifier leur aspect ou en corriger les défauts superficiels. Notamment, une composition cosmétique peut comprendre un véhicule cosmétiquement acceptable.

Par « véhicule cosmétiquement acceptable », on entend un véhicule adapté pour une utilisation en contact avec des cellules humaines cutanées, en particulier les cellules de l'épiderme, sans toxicité, irritation, réponse allergique indue et similaire, et proportionné à un rapport avantage/risque raisonnable. Le véhicule cosmétiquement acceptable peut être choisi parmi l'eau, la glycérine, l'allantoïne, le propanediol, cette liste n'étant pas limitative.

La composition cosmétique peut être obtenue par tout procédé approprié connu de l'homme du métier pour la fabrication d'une composition cosmétique. Il peut s'agir, par exemple d'un simple mélange.

La composition cosmétique peut en outre comprendre une ou plusieurs autres substances ou ingrédients actifs ou inactifs cosmétiquement. Les substances ou ingrédients inactifs sont ceux qui n'agissent pas cosmétiquement ou dermatologiquement. Il s'agit des éléments de la composition permettant notamment d'accompagner l'extrait, de constituer une formulation particulière, de conserver les extraits actifs dans le temps, etc. Il peut s'agir en outre de tout produit de base pouvant se trouver dans les compositions cosmétiques classiques. Par opposition, les substances ou ingrédients actifs sont ceux qui dans l'application cosmétique ou dermatologique visée ont une action esthétique et/ou médicale. Avantageusement, la composition cosmétique peut notamment comprendre un ou plusieurs agents anti-âge connus de l'homme du métier, autres que les extraits mentionnés ci-avant.

La composition cosmétique peut être sous forme de crème, de liquide ou de gel. Lorsque la composition est sous forme liquide, elle peut être choisie parmi un sérum, une lotion, un lait, une eau micellaire.

Alternativement, la composition cosmétique peut être sous forme de masque. Un tel masque peut comprendre un substrat insoluble dans l'eau imprégné par la composition cosmétique. Avantageusement, le substrat peut être en un matériau choisi parmi la bio-cellulose, l'hydrogel, les fibres de coton, les fibres de bambou, la cellulose, les microfibres, et les matériaux non tissé. Le masque peut avoir une épaisseur que l'homme du métier peut déterminer selon ses connaissances générales, en fonction du substrat choisi. Par exemple, si le masque est en bio-cellulose, il peut avoir une épaisseur comprise entre 0,15mm et 3,0 mm.

Avantageusement, le substrat peut être imprégné d'une quantité de composition cosmétique liquide adaptée pour pouvoir être au moins partiellement, voire totalement, imprégnée dans le masque, puis dans la peau. Il peut s'agir d'une quantité comprise entre 1 ml et 20 ml, par exemple 10 ml. Notamment, il peut s'agir d'un masque en bio-cellulose, imprégné d'environ 10ml de composition cosmétique sous forme de sérum.

L'étape de sophrologie est avantageusement une suite d'instructions données à l'utilisateur, par exemple au moyen d'un accompagnement audio, notamment pré-enregistré, et/ou de relaxation dynamique. Elle permet une application de la composition cosmétique en conscience, en présence, pour augmenter la biodisponibilité physique et mentale.

Selon l'invention, l'étape de sophrologie peut comprendre en outre les étapes suivantes :
(f) visualiser les effets cellulaires et cosmétiques du masque facial sur les cellules et la peau,
(g) se projeter après la séance en étant apaisée, ressourcée et ancrer ces sensations par la respiration.

Avantageusement, l'étape (c) permet de se concentrer sur la séance, en déconnectant le mental. Cette étape peut avoir une durée variable selon les individus, en fonction du besoin de l'individu. Elle peut par exemple être effectuée pendant une durée comprise entre 2 minutes et 10 minutes.

L'étape (d) peut avantageusement permettre d'installer une respiration régulière, une cohérence cardiaque, et ainsi une détente générale. La durée de cette étape peut varier en fonction du besoin de l'individu. Elle peut par exemple être effectuée pendant une durée comprise entre 2 minutes et 10 minutes.

L'étape (e) consiste à détendre chaque partie du corps de haut en bas pour atteindre un « état sophroliminal », entre la veille et le sommeil. Dans cet espace, l'imagination est plus forte que la volonté, il n'y a plus de freins, de peurs, de croyances limitantes. Ce qui est imaginé est intégré par l'organisme. La durée de cette étape peut varier en fonction du besoin de l'individu. Elle peut par exemple être effectuée pendant une durée comprise entre 2 minutes et 10 minutes.

L'étape de sophrologie peut en outre comprendre d'autres étapes, comme par exemple celle d'ancrer le calme et la sérénité, suite à l'étape (e). Cette étape permet avantageusement d'installer le calme dans l'esprit et le corps, de ressentir un calme profond. Il s'agit à l'individu de se projeter calme pour ancrer cette sensation et pouvoir la maintenir. La durée de cette étape peut varier en fonction du besoin de l'individu. Elle peut par exemple être effectuée pendant une durée comprise entre 2 minutes et 10 minutes.

Avantageusement, à l'issue de l'étape (e), l'individu est dans un état de sérénité et de connexion corps et mental, peau et esprit, qui permet, à l'étape (f), de sentir le sérum qui circule dans la peau, irrigue les cellules. Cette étape permet de booster la biodisponibilité du corps, de la peau, des cellules. La durée de cette étape peut varier en fonction du besoin de l'individu. Elle peut par exemple être effectuée pendant une durée comprise entre 2 minutes et 10 minutes, par exemple environ 3 minutes.

Le procédé de traitement cosmétique non thérapeutique de l'invention vise à améliorer l'esthétisme de la peau. Il s'agit donc d'une action purement esthétique, à l'exclusion de toute action thérapeutique, permettant d'améliorer l'aspect de surface de la peau, notamment pour présenter, à terme, un aspect plus esthétique, régulier et/ou homogène. On entend par « améliorer l'aspect de surface » tout effet permettant d'embellir, d'homogénéiser la partie visible de la peau et son aspect esthétique associé à des critères de jeunesse.

Ainsi, le procédé de l'invention peut être utilisé pour prévenir et/ou traiter le vieillissement cutané et/ou raffermir la peau et/ou hydrater la peau.

Dans le cadre des procédés cosmétiques selon l'invention, l'utilisation s'entend d'une utilisation non-thérapeutique, par exemple pour le traitement des peaux normales. Dans la présente, on entend par « peaux normales », des peaux ne présentant ou n'étant pas susceptible de présenter un état pathologique.

D'une manière générale, toute utilisation cosmétique et tout procédé cosmétique selon l'invention sont respectivement des utilisations cosmétiques non-thérapeutiques et procédés cosmétiques non-thérapeutiques.

D'autres avantages pourront encore apparaître à l'homme du métier à la lecture des exemples ci-dessous, illustrés par les figures annexées, donnés à titre illustratif.

### Brève description des figures

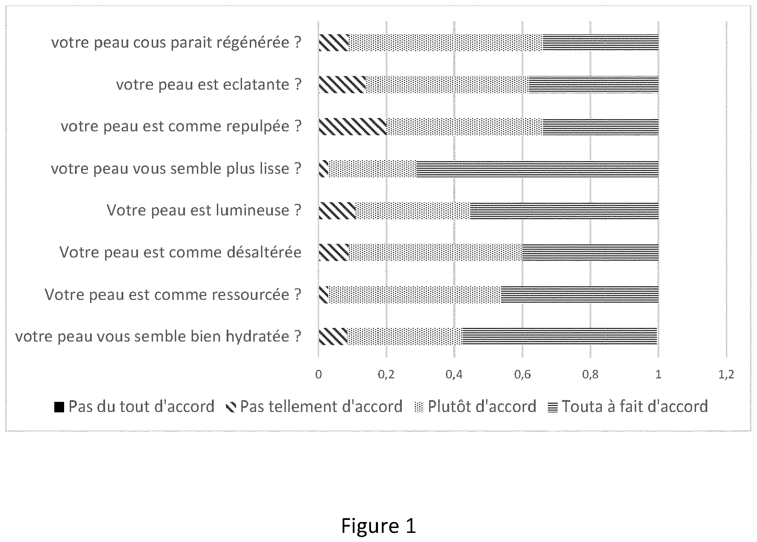

- La figure 1 représente un diagramme indiquant l'efficacité cutanée après utilisation du masque et de sa séance de sophrologie.

### EXEMPLES

### Exemple 1 : Mise en oeuvre du procédé de l'invention

Un masque visage en biocellulose naturelle est préparé, imprégnée de 10 ml de sérum, comprenant :
- 0,3% d'acide hyaluronique (Shandong Focus chem Biot ech Co., Ltd),
- 0,500% d'un extrait de fleur de Sophora Japonica (Résistress),
- 1% d'hydrolat de rose (PFVRELROSE14, Herbarom Laboratoire).

Les autres ingrédients du sérum sont les suivants : eau, glycérine, pentylène glycol, Polysorbate 20, propanediol, tétrasodium glutamate diacétate, parfum, acide citrique, géraniol, alcool benzylique, citronellol, hydroxyde de sodium et acide déhydroacétique.

Dans l'objectif d'accroître les bénéfices beauté et bien-être ressentis et visibles, le procédé de l'invention va, par les directives données aux utilisateurs/trices, permettre de travailler simultanément les 3 dimensions humaines suivantes :
- La dimension corporelle (détente physique / respiration / relaxation dynamique / les actifs dans la peau / les cellules),
- La dimension mentale (visualisation / stimulation mentale et biologique des effets sophroniques et cosmétiques)
- La dimension émotionnelle (projection et ancrage d'émotions positives liées au bien-être et à la beauté).

Les utilisateurs/trices suivent simplement les instructions données lors de la mise en oeuvre du procédé de l'invention, ce qui permet l'obtention des résultats décrits ci-après.

Le procédé de l'invention est mis en oeuvre sur 10 utilisatrices.

### Etape 1 : DECONNEXION - EVACUATION DES TENSIONS ET CENTRAGE SUR SOI : entre 6 et 8 min

- déconnecter le mental pour pouvoir se concentrer sur la séance

L'exercice consiste à décompter en respirant calmement pour sortir de ses pensées parasites
→ pour le Sophro Masque : 2min30
   - installer la respiration

L'exercice consiste à faire des respirations abdominales, respiration fondamentale pour se détendre car connectée au système nerveux parasympathique. Il est indiqué un rythme de 5 secondes à l'inspiration et de 5 secondes à l'expiration pour installer la cohérence cardiaque.
→pour le Sophro Masque : 3min30

### Etape 2 - RESPIRATION - DETENTE CORPORELLE ET MENTALE - ETAT SOPHROLIMINAL : entre 5 min et 7 min

- détendre l'ensemble du corps de la tête au pied / body scan

L'exercice consiste à détendre chaque partie du corps de haut en bas pour atteindre un « état dit sophroliminal », entre la veille et le sommeil. Dans cet espace, l'imagination est plus forte que la volonté, il n'y a plus de freins, de peurs, de croyances limitantes. Ce qui est imaginé est intégré par l'organisme.
→ pour le Sophro Masque : 4min30
   - ancrer le calme et la sérénité

Installer le calme dans son esprit et dans son corps, se sentir profondément calme. Se projeter calme pour ancrer cette sensation et pouvoir la maintenir.
--> pour le Sophro Masque : 1min40

### Etape 3 - VISUALISATION ET PROJECTION : entre 5 min et 8 min

- visualiser les effets cellulaires et cosmétique du sérum sur les cellules et la peau

Dans cet état de sérénité et de connexion corps et mental, peau et esprit, sentir le sérum qui circule dans la peau, irrigue les cellules. Booster la biodisponibilité de son corps, sa peau, ses cellules pour accueillir les bienfaits réhydratant et anti-stress de ce sérum
→ pour le Sophro Masque : 3min
   - se projeter après la séance en étant apaisée, ressourcée et ancrer ces sensations par la respiration
→ pour le Sophro Masque : 2min

Les effets du masque couplé à la séance sont mesurés à l'aide d'un test d'usage réalisé sur 35 personnes, en auto-évaluation au moyen d'un questionnaire précis, validée par une pharmacienne, sont indiqués en figure 1, et peuvent être résumés de la manière suivante :
- 97% des volontaires trouvent que leur peau est plus lisse
- 97% des volontaires trouvent que leur peau est ressourcée
- 91% des volontaires trouvent que leur peau parait régénérée
- 80% des volontaires trouvent que leur peau est repulpéeDes résultats comparatifs ont également été obtenus afin de démontrer l'efficacité, notamment cutanée, du masque couplé à la séance vs. l'efficacité du masque seul ou l'efficacité de la séance de sophrologie seule, et sont mentionnés dans le tableau I ci-après :

**Tableau I :**

| | Sans masque | Avec masque | Sans masque | Avec masque |
|---|---|---|---|---|
| | Sans séance de sophrologie | Sans séance de sophrologie | Avec séance de sophrologie | Avec séance de sophrologie |
| Traits du visage détendus | = | = | + | +++ |
| Peau désaltérée | = | ++ | = | +++ |
| Mental apaisé | = | = | ++ | +++ |
| Peau repulpée | = | ++ | = | +++ |
| Rides | = | 6% de rides en moins | = | 12% de rides en moins |
| Esprit libéré des tensions | = | = | ++ | +++ |
| Se sentir profondément et visiblement ressourcée | = | ++ | + | +++ |
| Se sentir profondément et visiblement calme | = | + | ++ | +++ |

| | | | | |
|---|---|---|---|---|
| Légende : = : pas de résultats constatés + : peu de résultats constatés ++ : quelques résultats constatés +++ : résultats constatés | | | | |

Les mesures du nombre de rides ont été réalisées avec le système in-vivo à projection de franges AEVA-HE (Eotech)

## Revendications

1. Procédé de traitement cosmétique non thérapeutique comprenant une étape d'application sur la peau du visage d'une composition cosmétique et au moins une étape de sophrologie, ladite composition cosmétique comprenant :
- de l'acide hyaluronique,
- un extrait de fleur de Sophora Japonica, et
- optionnellement un hydrolat de rose,
dans lequel l'étape de sophrologie comprend les étapes successives suivantes :
(a) adopter une position corporelle allongée ou semi-allongée,
(b) appliquer la composition cosmétique sous forme de masque sur le visage,
(c) décompter en respirant,
(d) réaliser des respirations abdominales, selon un rythme de 5 secondes à l'inspiration et de 5 secondes à l'expiration,
(e) détendre chaque partie du corps de haut en bas pour entrer dans un « état sophroliminal ».

2. Procédé selon la revendication 1, dans lequel l'étape de sophrologie comprend en outre les étapes suivantes :
(f) visualiser les effets cellulaires et cosmétiques du masque facial sur les cellules et la peau,
(g) se projeter après la séance en étant apaisée, ressourcée et ancrer ces sensations par la respiration.

3. Procédé selon l'une quelconque des revendications précédentes, dans lequel, en pourcentage en poids par rapport au total poids de la composition cosmétique :
- le pourcentage en poids d'acide hyaluronique est compris entre 0,01 % et 10,0%,
- le pourcentage en poids de l'extrait de fleur de Sophora Japonica est compris entre 0,1% et 5,0%.
- lorsque l'hydrolat de rose est présent, son pourcentage en poids d'hydrolat de rose est compris entre 0,01% et 50,0%.

4. Procédé selon l'une quelconque des revendications précédentes, dans lequel la composition cosmétique est sous forme de crème, de liquide ou de gel.

5. Procédé selon la revendication 3, dans lequel lorsque ladite composition est sous forme liquide, elle est choisie parmi un sérum, une lotion, un lait, une eau micellaire.

6. Procédé selon l'une quelconque des revendications 1 à 3, dans lequel la composition cosmétique est sous forme de masque.

7. Procédé selon la revendication 6, dans lequel ledit masque comprend un substrat insoluble dans l'eau imprégné par la composition cosmétique.

8. Procédé selon la revendication 7, dans lequel ledit substrat est en un matériau choisi parmi la bio-cellulose, l'hydrogel, les fibres de coton, les fibres de bambou, la cellulose, les microfibres et les matériaux non tissé.

9. Procédé selon l'une quelconque des revendications 7 ou 8 précédentes, dans lequel ledit substrat est imprégné d'une quantité de composition cosmétique liquide telle que définie dans la revendication 4 comprise entre 1 ml et 20 ml.

10. Procédé selon l'une quelconque des revendications précédentes, **caractérisé en ce que** le procédé est utilisé pour prévenir ou traiter le vieillissement cutané et/ou raffermir la peau et/ou hydrater la peau.

## Patentansprüche

1. Nicht-therapeutisches kosmetisches Behandlungsverfahren, das einen Schritt des Auftragens einer kosmetischen Zusammensetzung auf die Gesichtshaut und mindestens einen Schritt der Sophrologie umfasst, wobei die kosmetische Zusammensetzung umfasst:
- Hyaluronsäure,
- einen Extrakt der Blüte von Sophora Japonica, und
- gegebenenfalls ein Rosenhydrolat,
wobei der Schritt der Sophrologie die folgenden aufeinanderfolgenden Schritte umfasst:
(a) Einnehmen einer liegenden oder halb liegenden Körperposition,
(b) Auftragen des kosmetischen Mittels als Maske auf das Gesicht,
(c) Zählen beim Atmen,
(d) Bauchatmung mit einem Rhythmus von 5 Sekunden ein und 5 Sekunden aus,
(e) Entspannung jedes Körperteils von oben nach unten, um in einen "sophroliminalen Zustand" zu gelangen.

2. Verfahren nach Anspruch 1, wobei der Sophrologieschritt außerdem die folgenden Schritte umfasst:
(f) Visualisierung der zellulären und kosmetischen Wirkungen der Gesichtsmaske auf Zellen und Haut,
(g) sich nach der Sitzung zu projizieren, indem man sich beruhigt, gestärkt und diese Empfindungen durch die Atmung verankert.

3. Verfahren nach einem der vorangehenden Ansprüche, **dadurch gekennzeichnet, dass** als Gewichtsprozent, bezogen auf das Gesamtgewicht der kosmetischen Zusammensetzung
- der Gewichtsprozentsatz der Hyaluronsäure zwischen 0,01 % und 10,0 % liegt,
- der Gewichtsprozentanteil des Sophora Japonica-Blütenextrakts zwischen 0,1 % und 5,0 % liegt.
- wenn Rosenhydrolat vorhanden ist, liegt der Gewichtsprozentsatz des Rosenhydrolats zwischen 0,01 % und 50,0 %.

4. Verfahren nach einem der vorhergehenden Ansprüche, wobei die kosmetische Zusammensetzung in Form einer Creme, Flüssigkeit oder eines Gels vorliegt.

5. Verfahren nach Anspruch 3, wobei die Zusammensetzung, wenn sie in flüssiger Form vorliegt, ausgewählt ist aus einem Serum, einer Lotion, einer Milch, einem Mizellenwasser.

6. Verfahren nach einem der Ansprüche 1 bis 3, wobei die kosmetische Zusammensetzung in Form einer Maske vorliegt.

7. Verfahren nach Anspruch 6, wobei die Maske ein wasserunlösliches Substrat umfasst, das mit der kosmetischen Zusammensetzung imprägniert ist.

8. Verfahren nach Anspruch 7, wobei das Substrat aus einem Material besteht, das ausgewählt ist aus Bio-Cellulose, Hydrogel, Baumwollfasern, Bambusfasern, Cellulose, Mikrofasern und Vliesstoffen.

9. Verfahren nach einem der vorhergehenden Ansprüche 7 oder 8, wobei das Substrat mit einer Menge der flüssigen kosmetischen Zusammensetzung nach Anspruch 4 von 1 ml bis 20 ml imprägniert wird.

10. Verfahren nach einem der vorangehenden Ansprüche, **dadurch gekennzeichnet, dass** das Verfahren zur Vorbeugung oder Behandlung der Hautalterung und/oder zur Straffung der Haut und/oder zur Befeuchtung der Haut verwendet wird.

## Claims

1. A non-therapeutic cosmetic treatment process comprising a step of applying a cosmetic composition to the skin of the face and at least one step of sophrology, said cosmetic composition comprising:
- hyaluronic acid,
- an extract of Sophora Japonica flower, and
- optionally a rose hydrolat,
in which the sophrology step comprises the following successive steps:
(a) adopting a reclining or semi-reclining body position,
(b) applying the cosmetic composition as a mask to the face,
(c) counting while breathing,
(d) taking abdominal breaths, with a rhythm of 5 seconds in and 5 seconds out,
(e) relaxing each part of the body from top to bottom to enter a "sophroliminal state".

2. Process of claim 1, wherein the sophrology step further comprises the following steps:
(f) visualising the cellular and cosmetic effects of the face mask on cells and skin,
(g) projecting oneself after the session by being soothed, resourced and anchoring these sensations by breathing.

3. Process according to any one of the preceding claims, wherein, as a percentage by weight based on the total weight of the cosmetic composition:
- the percentage by weight of hyaluronic acid is comprised between 0.01% and 10.0%,
- the percentage by weight of the Sophora Japonica flower extract is comprised between 0.1% and 5.0%.
- when rose hydrolat is present, its percentage by weight of rose hydrolat is comprised between 0.01% and 50.0%.

4. Process according to any one of the preceding claims, wherein the cosmetic composition is in the form of a cream, liquid or gel.

5. Process according to claim 3, wherein when said composition is in liquid form, it is selected from a serum, a lotion, a milk, a micellar water.

6. Process according to any one of claims 1 to 3, wherein the cosmetic composition is in the form of a mask.

7. Process of claim 6, wherein said mask comprises a water-insoluble substrate impregnated with the cosmetic composition.

8. Process according to claim 7, wherein said substrate is of a material selected from bio-cellulose, hydrogel, cotton fibres, bamboo fibres, cellulose, microfibres and non-woven materials.

9. Process according to any one of the preceding claims 7 or 8, wherein said substrate is impregnated with an amount of liquid cosmetic composition as defined in claim 4 of between 1 ml and 20 ml.

10. Process according to any one of the preceding claims, **characterised in that** the process is used to prevent or treat skin ageing and/or firm the skin and/or moisturise the skin.
